# EUROPEAN PATENT APPLICATION

(11) **EP 2 520 332 A1**
(43) Date of publication of application: **07.11.2012**
(21) Application number: 11003672.0
(22) Date of filing: 05.05.2011
(51) Int. Cl.: A61N 1/378, A61N 1/39, A61N 1/375

(54) **Implantable stimulation device for defribillation and pacing**

(71) Applicant: Magnetic Pacing Technologies GmbH, 47057 Duisburg (DE)
(72) Inventor: Jung, Peter, 67697 Otterberg (DE); Kocks, Christian, 45481 Mülheim an der Ruhr (DE); Bai, Zijan, 47057 Duisburg (DE); Rickers, Sebastian, 46047 Oberhausen (DE); Bruck, Guido, 46562 Voerde (DE)
(74) Representative: Häckel, Stefan

(57) **Abstract**

The present invention relates to an implantable stimulation device for generating electrical stimulation impulses, wherein the stimulation device is adapted to be controlled and/or supplied with energy in an exclusively wireless manner by means of a time-varying magnetic field which can select high-energy and low-energy electrical impulses for defibrillation and pacing, respectively, based on different transmission characteristics of the time-varying magnetic field.

## Description

The present invention relates to an implantable stimulation device, preferably a cardiac defibrillator, to a stimulation system and to a method for controlling a stimulation device.

In the following description of the invention, the focus is primarily on cardiac defibrillators and/or pacemakers. However, the present invention is not restricted to this particular solution, but in general can be applied to other stimulation devices or systems which operate electrically and, in particular, deliver electrical impulses for stimulation. Alternatively or additionally, the stimulation device, the stimulation system and/or the method according to the present invention can be applied to nerve stimulation, muscle stimulation as well as to different fields of technology as well. For instance, the method according to the present invention can be applied to wireless electrolysis or electroanalysis of samples as well.

Defibrillators are devices delivering electrical energy to a heart such that its tissue is depolarized which stops malfunctions like cardiac arrhythmias, ventricular fibrillation and pulseless ventricular tachycardia. In particular, a strong electrical impulse applied to the heart can be used for defibrillation.

Cardiac pacemakers are used for regulating or re-synchronizing the beating of the heart. For pacemaking, electrical impulses can be applied to the heart as well. However, the amount of energy applied to the heart for defibrillation is much higher compared to that for pacemaking.

For providing electrical impulses to a heart, defibrillators and pacemakers can be implanted, for example, near the shoulder of the thoracic cage, at least one probe or electrical lead being guided from the implanted cardiac pacemaker or defibrillator via a vein into the atrium or the chambers of the heart and anchored there. The electrical lead is problematical and disadvantageous. It runs over a length of about 30 cm in the blood circulation system and can thereby cause undesirable or even fatal physical reactions. Furthermore, the risk of failure of the probes or leads due to material fatigue as a result of the mechanical stressing during body movements is particularly high. Another complication frequently encountered is dislocation of the probes triggered by movements of the patient.

US 2009/0024180 A1 discloses a stimulation system comprising an implantable electrode device, which is supplied with energy and controlled in an exclusively wireless manner via a time-variable magnetic field generated by an implanted control device. Thus, using an electrical lead is omitted and replaced by a wireless connection. The electrode devices are passive, which enables a compact size. However, these electrode devices will not be suitable for providing an electrical impulse with sufficiently high energy to perform defibrillation. In particular its small dimensions do not allow for depolarization of a sufficient share of muscle tissue, e.g. at least 70 % for defibrillation.

US 7,751,881 B2 relates to an acoustically-powered wireless defibrillator. An elongate defibrillation device is placed inside a heart extending from the apex of the right ventricle through the right atrium and into the superior vena cava. The defibrillator is charged acoustically over a short time for providing a pacing pulse and over a longer time for providing a defibrillation pulse. However, using acoustic power transmission requires reliable contact of the respective transmitter and receiver via a preferably non-compressible medium. Thus, a transmitter for acoustical energy needs to be coupled using ultrasonic gel or placed inside the body. Further, acoustical transduces are mechanical elements that are subject of wear due to rubbing as well.

Object of the present invention is to provide an implantable stimulation device, a stimulation system and a method for controlling a stimulation device, which are more reliable, more universally applicable and/or allow for more reliable or easier control.

This object is achieved by an implantable stimulation device according to claim 1, by a stimulation system according to claim 11 or by a method according to claim 15. Advantageous embodiments are subject of the subclaims.

The present invention provides an implantable stimulation device for generating electrical stimulation impulses, wherein the stimulation device is adapted to be controlled and/or supplied with energy in an exclusively wireless manner by means of a time-varying magnetic field. Thus, using mechanical transducers which are subject of wear is omitted. Further, energy or control commands can be transmitted without a direct and incompressible transmission medium and even without any direct contact.

According to a first aspect of the present invention, the stimulation device is configured for generating high-energy electrical impulses for defibrillation and low-energy electrical impulses for pacing. A high-energy electrical stimulation impulse is generated when the stimulation device is supplied with energy or controlled by means of the time-varying magnetic field of a first transmission characteristic, in particular of a first frequency. A low-energy electrical stimulation impulse is generated when the stimulation device is supplied with energy or controlled by means of the time-varying magnetic field of a different second transmission characteristic, in particular a different second frequency.

This allows for a reliable control and activation of the different modi for defibrillation and pacing. Pacing can be activated while the stimulation device is charged for defibrillation. Further, transmission characteristics can be used for controlling the stimulation device, in particular high-energy and/or low-energy modi, with little effort and with low power, e.g., even using passive filtering structures without any control effort by the stimulation device. An electrical stimulation impulse, in the following referred to as stimulation impulse, preferably is a voltage, current and/or electrical power provided via electrodes to a tissue or sample for stimulating it.

A further aspect of the present invention, that can be realized independently as well, relates to a stimulation device comprising a multiplicity of linked elements, wherein at least two or all of the elements are adapted to store and/or to be supplied with energy by means of the time-varying magnetic field. This leads to a stimulation device, wherein means for storing and/or receiving energy are distributed.

Further, capacity and length of the stimulation device is adaptable to individual applications. In particular, the amount of energy to be stored and/or the density of energy that is transmittable can be configured individually by choosing a number of elements. For instance, in some cases a smaller area is to be stimulated, e.g., if a smaller heart is present. Then, a distance between two stimulation electrodes for defibrillation as well as the energy needed for defibrillation can be reduced. According to the present invention, the stimulation device can be reduced by some elements which allows for reducing the length of the stimulation device and the overall capacity for storing energy, simultaneously, leading to scalability of the stimulation device.

Preferably, at least two of the elements each comprises an energy storing means, preferably configured to store more than 50 mJ, in particular more than 100 mJ and/or less than 2 J, in particular less than 1J. This allows the stimulation device for storing energy sufficient for defibrillation.

Preferably, at least two of the elements each comprises a receiving means which is adapted for receiving energy in an exclusively wireless manner by means of the time-varying magnetic field and converts this energy to electrical energy, preferably with an output power rating of more than 50 mW, in particular more than 100 mW and/or less than 2 W, in particular less than 1 W, and/or wherein the stimulation device is adapted to be charged with more than 500 mW, preferably more than 1 W and/or less than 20 W, preferably less than 10 W. This enables the stimulation device to be charged in short term, in particular less than 5 s.

Preferably, the linked elements form a joint energy storing means and/or receiving means of the stimulation device, preferably wherein the amount of energy storable by the stimulation device and/or the power rating of energy transferable to the stimulation device is or are variable by means of the number of elements the stimulation device comprises. Thus, the stimulation device can be adapted individually.

Preferably, the stimulation device is flexible by means of the multiplicity of linked elements, preferably wherein the elements are shaped as tube-like sections, and/or wherein face sides of adjacent elements are hinged to each other. This supports variable applicability.

Preferably, the multiplicity of linked elements are forming the preferably elongate shape of the stimulation device, in particular of a length greater than 10 cm, preferably greater than 12 cm, in particular greater than 15 cm and/or smaller than 25 cm, preferably smaller than 22 cm, in particular smaller than 18 cm, and/or of a diameter smaller than 5 mm, preferably smaller than 4 mm, in particular smaller than 3 mm, and/or greater than 0.5 mm, preferably greater than 1 mm, in particular greater than 1.5 mm.

Preferably, the stimulation device provides a high-energy mode and a low-energy mode, wherein the energy delivered by the stimulation device for each stimulation in the high-energy mode is higher than energy delivered for each stimulation in the low-energy mode, preferably at least the 100 times, in particular 200 times, higher. Thus, defibrillation and/or cardiac pacing can be supported.

Preferably, the stimulation device is adapted to selectively activate the high-energy or the low-energy mode, respectively, if energy is transmitted using a time-varying magnetic field of a first or a different second transmission characteristic, preferably of a first and a different second frequency.

Preferably, the stimulation device comprises an anchor for securely fastening it which is adapted for delivering an electrical impulse additionally.

Preferably, the stimulation device comprises a first pair of electrodes having a shorter distance to one another which is configured to deliver low-energy impulses, in particular pacing impulses and/or to a tissue of a cardiac muscle, and a second pair of electrodes having a larger distance to one another which is configured to deliver high-energy impulses, preferably defibrillation impulses and/or to a tissue of a cardiac muscle. Thus, the low-energy stimulation impulse can be delivered more efficiently and/or the high-energy stimulation impulse reaches a sufficient large area.

A further aspect of the present invention, that can be realized independently as well, relates to a stimulation system comprising a stimulation device adapted for delivering an electrical impulse, which preferably is configured according to one of the above aspects. Further, the stimulation system comprises a control device adapted for controlling the stimulation device and/or supplying the stimulation device with energy in an exclusively wireless manner by means of a time-varying magnetic field. The control device preferably is adapted to generate time-varying magnetic fields of different transmission characteristics. Thus, the control device can control and/or supply one more stimulation devices in a wireless manner, regardless of whether it is implanted, attached to a body or not. This leads to increased flexibility in application of the stimulation system.

Preferably, the control device and the stimulation device are adapted to transmit and receive energy by means of the time-varying magnetic field, respectively, which provides both energy and control information to the stimulation device sufficient for generation of the electrical impulse by the stimulation device, and/or with transfer power of more than 500 mW, preferably higher than 1 W and/or lower than 20 W, preferably lower than 10 W. This allows for charging the stimulation device in short term.

Preferably, the control device comprises sensing electrodes configured to detect cardiac arrhythmias, preferably wherein the control device is adapted to automatically transmit energy by means of time-varying magnetic field of a particular transmission characteristic, in particular frequency, when a cardiac arrhythmia is detected and the stimulation device is adapted to enter high-energy mode and/or to deliver a defibrillation impulse if the stimulation device receives energy by means of the time-varying magnetic field of this transmission characteristic, in particular frequency. This enables automatically activating generation and/or delivery of the stimulation impulse.

Preferably, the system further comprises an external programmer configured to program one or more settings of the control device, in particular corresponding to transmission characteristics, in particular frequencies, for the time-varying magnetic field to be used for entering high-energy mode and/or low-energy mode of the stimulation device. The control device can be implanted and the programmer can be adapted to program the control device in a wireless manner, in particular by means of the time-varying magnetic field. This allows the system to be adapted individually and/or subsequently.

A further aspect of the present invention, that can be realized independently as well, relates to a method for controlling a stimulation device for generating high-energy and low-energy stimulation impulses, wherein the stimulation device is controlled and/or supplied with energy in an exclusively wireless manner by means of a time-varying magnetic field, such that the stimulation device generates a high-energy electrical stimulation impulse when supplied with energy or controlled by means of a time-varying magnetic field of a first frequency and a low-energy electrical stimulation impulse when supplied with energy or controlled by means of a time-varying magnetic field of a different second frequency. Thus, defibrillation and pacing can be activated selectively and independently.

In the sense of the present invention, the term "magnetic field" preferably covers magnetic and electro-magnetic fields or waves. In particular, fields, waves or the like with any kind of magnetic component can be "magnetic fields" in the sense of the present invention. Furthermore, in the following "the magnetic field" is used in singular. However, the magnetic field can have different properties over time and in space, can comprise more than one (sub-)field and/or independent or superposed components and/or different transmission characteristics, in particular simultaneously or intermittingly.

In addition to one or more stimulation devices, one or more electrode devices for generation of further (low-energy) electrical stimulation impulses can be provided, which preferably have a compact size and a passive behaviour, in particular as described in US 2009/0024180 A1.

In the sense of the present invention, the term "time-varying magnetic field" preferably is related to one or more of the stimulation device or further electrode devices or their location, in particular in an implanted or ready-to-use arrangement or condition. This means, a particular field, field strength, geometry or variation of the time-varying magnetic field preferably relates to a position of the stimulation and/or electrode device. Furthermore, it is preferred that the "magnetic field" reaches and/or affects at least one, preferably each one, of the stimulation and/or electrode devices of the stimulation system. However, the time-varying magnetic field might affect different stimulation and/or electrode devices in different manner, intensities and/or with different influence or effect on different electrode devices.

In the sense of the present invention, "transmission characteristics" of a magnetic field preferably relate to a field strength, to a geometry of the field, to a variation over time of the field and/or to a position of one or more of the electrode devices. In particular, a transmission characteristic is a characteristic of and/or affects the time-varying magnetic field strength or geometry of the magnetic field at the location of the stimulation device(s) and/or electrode device(s). The term "transmission characteristic" of the magnetic field preferably covers any characteristic assigned to the magnetic field with distinguishable states. Particularly preferably, transmission characteristics cover frequencies and/or polarities as explained in the following. However, further transmission characteristics might be, for example, particular directions, directivities, angles of transmission, foci, beam forms and/or field patterns. In particular, different transmission characteristics can be achieved using beam forming techniques.

According to the present invention, transmission characteristics particularly preferably are (characteristic) frequencies and/or polarities of the magnetic field.

In the sense of the present invention, a "frequency" of a magnetic field preferably is a variation of the field strength over time and/or at a location of one of the stimulation and/or electrode devices. The magnetic field preferably has a significant amplitude at this frequency and/or comprises energy or is adapted to transfer energy at this frequency. For example, a significant amplitude can be greater than the thousand fold of the static magnetic field strength of the earth or can be greater than 100 µT, preferably greater than 1 mT, in particular greater than 5 or 10 mT.

According to the present invention, a "polarity" of a magnetic field can be related to a spatial characteristic of the magnetic field, in the following referred to as "spatial polarity" and/or to a temporal characteristic of the magnetic field, in the following referred to as "temporal polarity".

A "spatial polarity" of a magnetic field can be a field geometry or wave geometry, preferably a polarization or an orientation of oscillations and/or of field lines, in particular in a plane perpendicular to a direction of propagation, transmission, direction of travel, and/or to a transverse wave's direction of travel.

In particular, the spatial polarity can cover horizontal, vertical and/or circular polarizations or orientations of the magnetic field or its oscillations in a plane or the like, in the following referred to as "field polarity".

Alternatively or additionally, a spatial polarity according to the present invention relates to an orientation of a magnetic field source. In particular, a north and south pole of a magnetic field can be exchanged for changing the polarity. A polarity relating to different orientations of the magnetic poles of a magnetic field source in the following is referred to as "source polarity".

A "temporal polarity" of a magnetic field according to the present invention preferably is a polarity that relates to a particular time-variation of the magnetic field, in particular of the magnetic field strength and/or at the location of an electrode device.

A magnetic field can increase or decrease over time. An increasing field strength over time has a positive slope or gradient and/or a decreasing field strength over time has a negative slope or gradient. Hence, the magnetic field can have different polarities in the meaning of slopes or gradients over time, in particular of a varying field strength, with different signs, which in the following is referred to as "gradient polarity".

Alternatively or additionally, the magnetic field in addition to a time-variable component can comprise an, in particular at least temporarily, time-invariant component which is termed "offset". In particular, an offset is a static magnetic field that is added to the time-varying magnetic field. Thus, a static and a time-varying magnetic field component can be superposed. When added to the offset, the time-varying component of the magnetic field, even if it is alternating, does not need to change the source polarity. Hence, the magnetic field can have a polarity in the sense of an offset, which can have different signs, i.e. source polarities (orientations) and/or field strengths, in the following is referred to as "offset polarity".

Preferably, time-varying magnetic fields of different polarities cover or are time-varying magnetic fields of at least one of a spatial polarity, temporal polarity, field polarity, source polarity, gradient polarity and/or offset polarity.

In the sense of the present invention, a "negative magnetic field" or a "negative field strength" preferably is a magnetic field of an absolute value of a field strength, wherein poles are changed, i.e. a north pole is replaced by south pole and vice versa. If a magnetic field is generated by a current flow, e.g. through a coil, a negative field strength can correspond to and/or can be generated by inverting the direction of the current flow.

It is particularly preferred that different stimulation and/or electrode devices and/or modi of the stimulation device, in particular the high-energy and/or low-energy modi, can be controlled, triggered and/or supplied with energy independently. Then, electrical impulses can be generated at different times and/or intensities, i.e. with a particular time delay between generation of electrical impulses of the different stimulation and/or electrode devices. This allows for an improved synchronization of stimulation. In particular, a high-energy mode for delivering a defibrillation impulse and a low-energy mode for delivering a pacing impulse can be activated separately and/or simultaneously.

As already explained, the implantable stimulation device is used in particular for generating electrical signals or impulses to stimulate a heart, in particular via electrodes and/or by causing a current through the tissue of the heart. However, the present invention is not restricted to this. Rather, the stimulation device and/or the stimulation system can generally generate any type of electrical impulse or electrical signal, in particular in the human or animal body as well as for electrolysis, electroanalysis of samples or the like. The terms "stimulation device" and "stimulation system" should accordingly be understood in a very general sense so that other applications and uses can be understood.

The preceding is a simplified summary of the invention to provide an understanding of some aspects of the invention. This summary is neither an extensive nor exhaustive overview of the invention and its various embodiments. It is intended neither to identify key or critical elements of the invention nor to delineate the scope of the invention but to present selected concepts of the invention in a simplified form as an introduction to the more detailed description presented below. As will be appreciated, other embodiments of the invention are possible utilizing, alone or in combination, one or more of the features set forth above or described in detail below. In particular, different aspects of the present invention can be combined.

Further advantages, properties, features and aspects of the present invention are obtained from the claims and the following description of preferred embodiments with reference to the drawings.

In the figures:
- Fig. 1: is a schematic view of a stimulation system according to the present invention;
- Fig. 2: is a schematic view of a stimulation device according to the present invention;
- Fig. 3: is a simplified schematic view of an element of a stimulation device according to the present invention;
- Fig. 4: is a partial, sectional view of a stimulation device according to the present invention;
- Fig. 5: is a simplified schematic view of a stimulation device according to a further embodiment of the present invention;
- Fig. 6: is a simplified schematic view of a stimulation device according to a further embodiment of the present invention;
- Fig. 7: is a schematic view of a control device according to the present invention;
- Fig. 8: is a time diagram according to the present invention;
- Fig. 9: is a simplified schematic view of a stimulation system according to the present invention; and
- Fig. 10: is a time diagram according to the present invention.

In the figures the same reference numerals are used for the same parts or parts of the same type, components or the like, wherein corresponding or similar advantages and properties are obtained even if a description is not repeated. Furthermore, in the following reference is made to the definitions and explanations given above, in particular regarding transmission characteristics and various categories and implementations of different transmission characteristics. In the following, transmission characteristics mentioned in the beginning are represented by the particularly preferred frequency. However, different of the other transmission characteristics can be used additionally or alternatively.

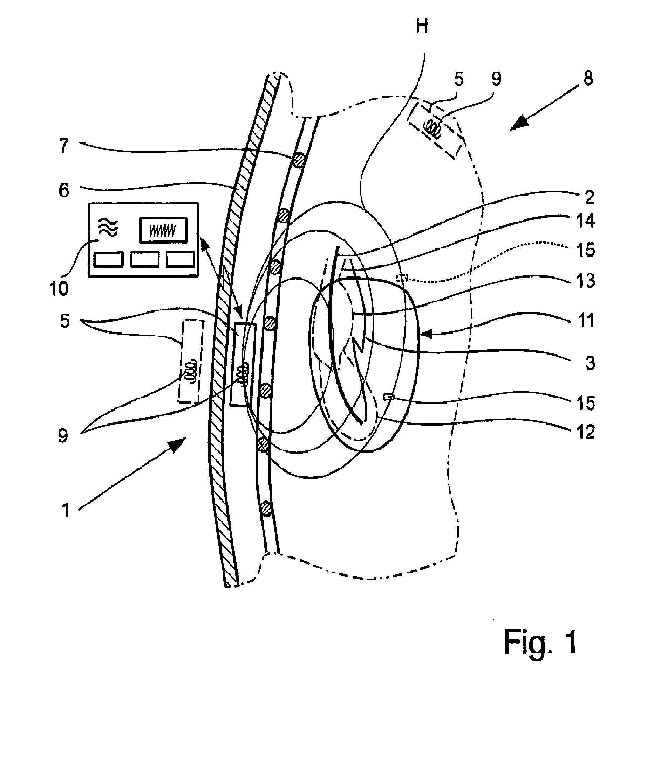

Fig. 1 is a schematic view of a proposed stimulation system 1 with a stimulation device 2 for generating electrical stimulation impulses 3, wherein the stimulation device 2 is adapted to be controlled and/or supplied with energy in an exclusively wireless manner by means of a time-varying magnetic field H.

The stimulation system 1 preferably is configured or works as a cardiac defibrillator and/or pacemaker in the example shown. However, the present invention is not restricted thereto. For example, the stimulation system 1 can additionally or alternatively operate as a wireless device for stimulating a sample material in electrolysis and/or electroanalysis as well. For instance, stimulation device 1 can be used for performing electroanalysis of a flowing liquid in a pipe and/or with at least two different currents corresponding to high-energy and low-energy modes.

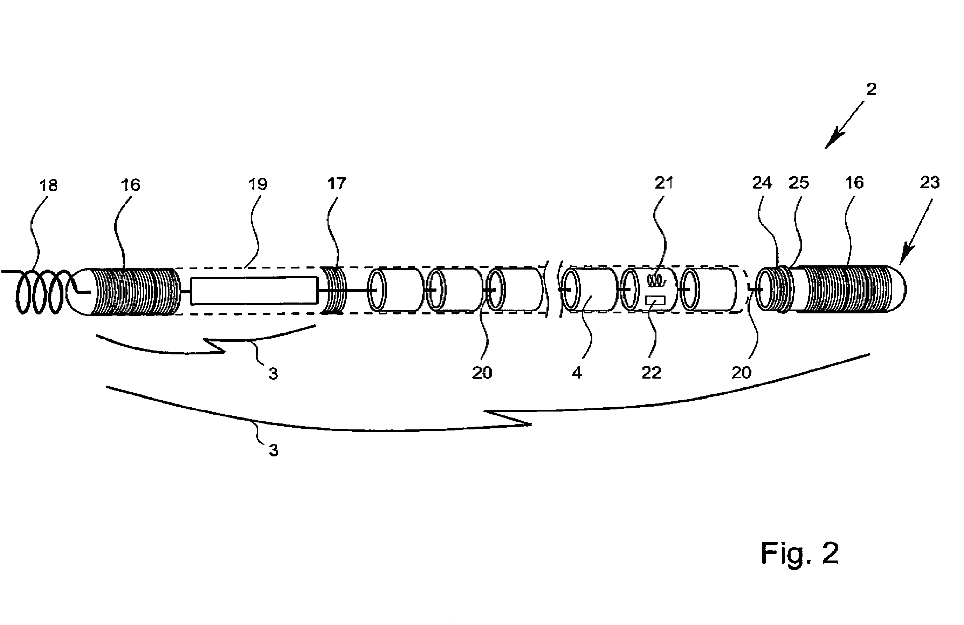

Stimulation device 2 preferably has an elongate shape. According to one aspect of the present invention, stimulation device 2 comprises a multiplicity of linked elements 4, wherein at least two or all of the elements 4 are adapted to store energy and/or to be supplied with energy by means of the time-varying magnetic field H which is discussed later in further detail referring to Fig. 2.

Stimulation system 1 in Fig. 1 comprises a control device 5. Control device 5 can be implanted between skin 6 and thoracic cage 7 of a body 8. Alternatively or additionally, control device 5 can be placed outside the body 8 or in the field of a shoulder, in particular as depicted using dashed lines.

Control device 5 is adapted for controlling stimulation device 2 and/or for supplying stimulation device 2 with energy each and/or both in an exclusively wireless manner by means of the time-varying magnetic field H. The time-varying magnetic field H preferably is generated by control device 5 using a sending means 9, in particular a coil. Control device 5 can be programmable by means of an external programmer 10. Control device 5 can be adapted for sensing electrical signals, in particular signals corresponding to an activity of a heart 11. This allows for adapting the time-varying magnetic field H by control device 5 depending on the present status of a heart 11.

Stimulation device 2 preferably is placed close to or inside heart 11. Stimulation device 2 extends from the right ventricle 12 of heart 11 through the right atrium 13 to the superior vena cova 14 in the example shown. However, different positions are possible as well.

Optionally, in addition to stimulation device 2 further electrode devices 13 can be placed close to or inside the heart. It is particularly preferred that stimulation system 1 is adapted for defibrillation as well as for pacing heart 11. In a first embodiment, defibrillation and pacing both can be carried out by stimulation device 2. Additionally or alternatively, one or more electrode devices 15 can be used for supporting or performing pacing heart 11.

Fig. 2 shows implantable stimulation device 2 according to an embodiment of the present invention. Stimulation device 2 preferably comprises at least two electrodes 16, in particular at distal ends of elongate stimulation device 2. Additionally or alternatively, stimulation device 2 can comprise one or more ring electrodes 17 and/or an anchor 18 which can be adapted for fastening stimulation device 2 and/or which can act as an electrode.

Stimulation device 2 further comprises cover 19, in particular a watertight casing, which preferably is biocompatible and allows for implanting stimulation device 2. Cover 19 in Fig. 2 is indicated using dashed lines. Cover 19 preferably is flexible and allows for bending stimulation device 2. For example, cover 19 can comprise synthetic materials, in particular silicone or the like.

Stimulation device 2 preferably has an elongate shape, in particular with a length of more than the 40-fold, preferably the 60-fold, in particular the 80-fold, of a diameter of stimulation device 2. It can have a longitudinal extension of more than 10 cm, preferably more than 12 cm, in particular more than 15 cm and/or less than 25 cm, preferably less than 22 cm, in particular less than 18 cm. A diameter of stimulation device 2 can be smaller than 5 mm, preferably smaller than 4 mm, in particular smaller than 3 mm, and/or greater than 0,5 mm, preferably greater than 1 mm, in particular greater than 1,5 mm. Moreover, the elongate shape of stimulation device 2 can be formed by the multiplicity of linked elements 4, preferably wherein elements 4 are linked flexibly, in particular by means of flexible interfaces 20.

Stimulation device 2 can be flexible by means of the multiplicity of linked elements 4, preferably wherein the elements 4 are shaped as stiff and/or tube-like sections and/or wherein face sides of adjacent elements 4 are hinged to each other. Adjacent elements 4 preferably are connected via interfaces 20, in particular providing a flexible mechanical and/or electrical interconnection.

It is further preferred that at least one, in particular each one, of the elements 4 comprises a receiving means 21, in particular a coil, and/or an energy storing means 22, in particular a capacitor or rechargeable battery. In particular, at least two of the elements 4 each comprises an energy storing means 22, preferably configured to store more than 50 mJ, in particular more than 100 mJ and/or less than 2 J, in particular less than 1 J. It is further preferred that at least two of the elements 4 each comprises a receiving means 21 which is adapted for receiving energy in an exclusively wireless manner by means of the time-varying magnetic field H. Further it is preferred that the receiving means 21 converts this energy to electrical energy, preferably with an output power rating of more than 50 mW, in particular more than 100 mW and/or less than 2 W, in particular less than 1 W each.

The energy storing means 22 of some or all of the elements 4 can be connected forming a joint energy storing means 22 of the stimulation device 2, in particular by means of interfaces 20. Stimulation device 2 preferably comprises an overall capacity of more than 1 J, preferably more than 2 J, in particular more than 5 J and/or less than 30 J, preferably less than 20 J, in particular less than 15 J. This enables generation of a stimulation impulse 3 which is sufficient for defibrillation, in particular for depolarization of at least 70 % of heart 11. It is further preferred that stimulation device 2 is adapted to be charged with more than 500 mW, preferably more than 1 W and/or less than 20 W, preferably less than 10 W. This allows for charging stimulation device 2 with an amount of energy which is sufficient for fast defibrillation. Preferably, stimulation device 2 can be sufficiently charged for defibrillation in less than 5 s, in particular less than 3 s, which allows for quick reaction if defibrillation becomes necessary.

According to one aspect of the present invention, the amount of energy storable by stimulation device 2 and/or the power rating of energy transferable to stimulation device 2 is or are variable by means of the number of elements 4 the stimulation device 2 comprises. In particular, stimulation device 2 as shown in fig. 2 comprises a multiplicity of linked elements 4, for instance more than 10, preferably 15 and/or less than 40, preferably less than 30 and/or in a row forming a chain or string, in particular corresponding to the elongate shape of stimulation device 2. Moreover, each element 4 can comprise receiving means 21 and/or energy storing means 22. Thus, the joint capacity of stimulation device 2 and/or the maximum power that can be transferred to stimulation device 2 can be varied by choosing the number of elements 4 to be used forming stimulation device 2.

In the example shown, elements 4 of stimulation device 2 can be removed and cover 19 of stimulation device 2 can be closed afterwards by means of a cap 23, preferably shown comprises one of the electrodes 16 and/or of the interfaces 20. However, cap 23 does not necessarily comprise an electrode 16 but also can be implemented for closing cover 19 only or can comprise different functions alternatively or additionally, e.g. receiving or control means. Cap 23 can comprise a mounting means, in particular thread 24, for mounting the cap and securely and/or watertight fastening cap 23 at cover 19, preferably closing cover 19. A seal can be applied to cap 23, in particular by means of a circumferential beat 25, which preferably allows for sealing cap 23 against cover 19.

A controller 24 can be used for generating the stimulation impulse 3, in particular wherein a voltage is applied to the electrodes 16, 17. In particular, a stimulation impulse 3 for pacing can be applied to ring electrode 17 and to at least one of the electrodes 16, in particular by means of a voltage pulse. Alternatively or additionally, a stimulation impulse 3 for defibrillation is preferably applied (to tissue 2) by means of a voltage pulse between the electrode 16 at the distal ends of stimulation device 2.

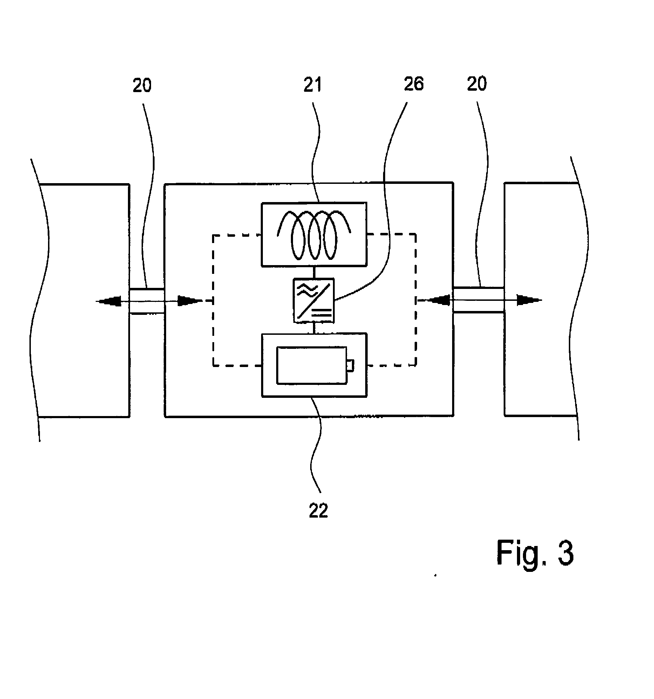

Fig. 3 shows a simplified schematic view of one of the linked elements 4. Element 4 preferably is adapted to receive energy by means of time-varying magnetic field H with receiving means 21, preferably a coil. Receiving means 21 preferably can convert energy transmitted by means of the time-varying magnetic field H to electrical energy. This electrical energy can be rectified and/or controlled by a charging means 26, which preferably is adapted to control the charging progress of energy storing means 22. In particular, charging means 26 can comprise or can be formed by one or more diods, a rectifier, a current limiter, voltage limiter, and/or controller. Each of the elements 4 can comprise at least one interface 20, preferably two interfaces 20 at opposing ends of element 4.

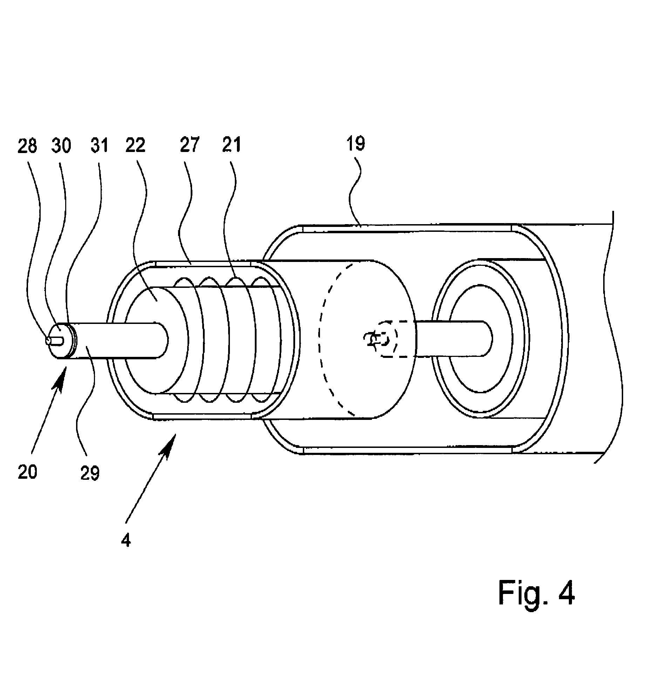

Fig. 4 shows a partial cross sectional view of stimulation device 2. Element 4 in Fig. 4 is shaped as a tube-like section. In the example shown, element 4 has a circumferential cross section. However, different shapes can be used as well, even if an at least basically eliptical cross section is preferred since it supports a comfortable corrsponding cross sectional shape of stimulation device 2.

An optional outer shell 27 of element 4 comprises energy storing means 22, in particular a tubular capacitor, which can be surrounded by receiving means 21, in particular windings of a respective coil. Interface 20 in preferably comprises an inner lead 28 and an outer lead 29, in particular separated by insulator 30. Interface 20 preferably is adapted to electrically and mechanically link adjacent elements 4 to each other. In particular, interface 20 provides a mechanically flexible interconnection, preferably wherein interface 20 is assigned or fixed to one of the elements 4 and can be mounted, in particular screwed, on an adjacent one of the elements 4. Interface 20, in particular outer lead 29, comprises a mounting means 31, in particular for locking and/or screwing two of the elements 4 together. Thus, two adjacent elements 4 can be electrically and/or mechanically connected and/or disconnected by means of interface 20.

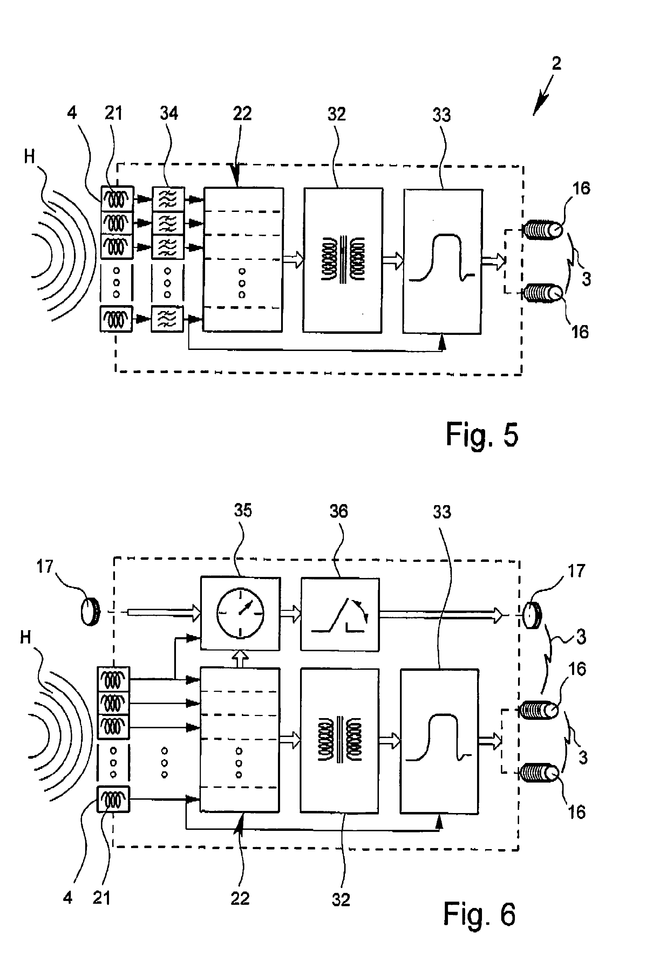

Fig. 5 shows a schematical block diagram of a stimulation device 2 according to one embodiment of the present invention. Stimulation device 2 is adapted to receive energy by means of time-varying magnetic field H. In particular, receiving means 21 of multiple elements 4 are used for receiving energy and, preferably, for converting it to electrical energy. This energy can be stored to energy storing means 22 which can be separate for each of the elements 4 or can be formed by a common energy storing means 22 for several or all of the elements 4.

The energy stored in energy storing means 22 can be converted to a stimulation impulse 3 by a converter 32 and can be delivered to electrodes 16, optionally via a pulse-forming means 33. In particular, converter 32 allows for generating a high-voltage stimulation impulse 3 which can be used for defibrillation. For example, converter 32 can be adapted to generate voltages higher than 100 V, preferably 200 V, in particular 400 V. Pulse-forming means 33 can be used for reshaping the stimulation impulse 3, preferably in the time domain and/or by limiting the voltage or current provided to electrodes 16,17. Pulse-forming means 33 can be adapted to be triggered such that stimulation impulse 3 is delivered at a specific time. This triggering can be controlled by means of the time-varying magnetic field H, preferably by using a specific transmission characteristic, in particular frequency.

In the example shown in Fig. 5, filtering means 34 can be used such that energy storing means 22 is charged only and/or stimulation impulse 3 is delivered via electrodes 16, 17 only if time-varying magnetic field H has a specific transmission characteristic, in particular a frequency inside the pass-band of filtering means 34. Receiving means 21 can be adapted such that the function of filtering means 34 is realized by receiving means 21. For example, coils can be used as receiving means 21 which are configured as resonant circuits realizing filtering means 34. However, different solutions may exist. In particular, a frequency can be detected and/or used for triggering pulse-forming means 33 which is indicated by an arrow in Fig. 5.

Stimulation device 2 preferably provides a high-energy mode and a low-energy mode, in particular wherein stimulation device 2 provides a stimulation impulse 3 sufficient for defibrillation in the high-energy mode and/or wherein stimulation device 2 provides energy sufficient for pacing and/or less than the amount of energy sufficient for defibrillation in low-energy mode. This allows stimulation device 2 for providing either defibrillation or pacing.

Stimulation impulse 3 for defibrillation and/or in the high-energy mode preferably provides energy of more than 1 J, preferably more than 2 J, in particular more than 5 J. Stimulation impulse for cardiac pacing and/or in the low-energy mode preferably provides energy of more than 100 nJ, preferably more than 200 nJ, in particular more than 500 nJ and/or less than 1 J, preferably less than 100 mJ, in particular less than 10 mJ. A stimulation impulse for defibrillation and/or in the high-energy mode preferably provides more than 100 times, in particular more than 200 or 500 times, the energy provided by a stimulation impulse 3 for pacing and/or in the low-energy mode.

Stimulation device 2 preferably is adapted to selectively activate the high-energy or low-energy mode, respectively, if energy is transmitted using a time-varying magnetic field H of a first or a different second transmission characteristic, preferably of a first and a different second frequency. In the embodiment shown in Fig. 5, the same electrodes 16 can be used in the high-energy and in the low-energy mode. However, different electrode configurations can be possible. For example, different electrodes 16, 17 are used in the high-energy and in the low-energy modes. Alternatively or additionally, at least one electrode 16, 17 can be jointly used in the high-energy and in the low-energy mode, preferably wherein at least one further electrode 16, 17 is used in the high-energy mode or in the low-energy mode only. Thus, for defibrillation and pacing at least one joint electrode 16,17 can be used.

Fig. 6 shows stimulation device 2 according to a further embodiment. A repeated discussion of the same or similar parts is not repeated. However, the same or similar properties or advantages are achieved, in particular as discussed referring to Fig. 5. Features or components of embodiments shown in Fig. 5 and 6 can be combined.

Receiving means 21 of a multiplicity of elements 4 can be used for receiving energy by means of time-varying magnetic field H. However, additional and/or one or more joint receiving means 21 can be used as well. The received energy can be stored in energy storing means 22, which can be formed by elements 4 and/or separate therefrom. Converter 32 can be used for forming stimulation impulse 3 from electrical energy stored in energy storing means 22. Pulse forming means 33 can be used for reforming a stimulation impulse 3, in particular a duration and/or amplitude of stimulation impulse 3 which, preferably, can be delivered via electrodes 16, 17. Further, stimulation device 2 can comprise a triggering means 35 and/or an activation means 36, in particular a switch.

In the example shown, electrodes 16 preferably are used for delivering high-energy stimulation impulse 3. One or more electrodes 17 can be used for delivering low-energy stimulation impluse 3, e.g. for pacing. Delivery of low-energy stimulation impulse 3 preferably is synchronized by stimulation device 2, in particular to a sample status or to the electrical activity of heart 11.

Stimulation device 2 in Fig. 6 can be adapted to synchronize generation and/or delivery of low-energy stimulation impulses 3 with triggering means 35. In particular, triggering means 35 can receive information via an electrode 17, e.g. from the surrounding tissue, and/or by means of the time-varying magnetic field H, in particular by means of its transmission characteristic.

According to one aspect of the present invention, providing low-energy stimulation impulses 3 can be activated using a time-varying magnetic field H of a first transmission characteristic and sychronization can be performed based on time-varying magnetic field H and/or based on signals received via an electrode. Triggering means 35 in particular can delay generation and/or delivery of a low-energy stimulation impulse 3. Triggering means 35 can be used for controlling activation means 36 to deliver a low-energy stimulation impulse 3. Alternatively or additionally, providing low-energy stimulation impulses 3 can be activated and synchronized both by means of time-varying magnetic field H.

High-energy stimulation impulses 3 can be synchronized as well. However, delivering high-energy stimulation impulses 3 often are used if synchronization is not necessary or possible, in particular in case of ventricular fibrillation or tachycardia. Therefore, stimulation device 2 in Fig. 6 does not provide triggering means for delivering high-energy impulses 3.

Stimulation device 2 can be adapted to be controlled by means of different transmission characteristics of time-varying magnetic field H. For example, delivering a high-energy stimulation impulse can be activated by transmitting energy using a time-varying magnetic field H of a first frequency. Alternatively or additionally, a time-varying magnetic field H of a different frequency can be used for activating delivery of low-energy stimulation impulses. These low-energy stimulation impulses can additionally be triggered by means of a sensed signal and/or by means of the time-varying magnetic field H.

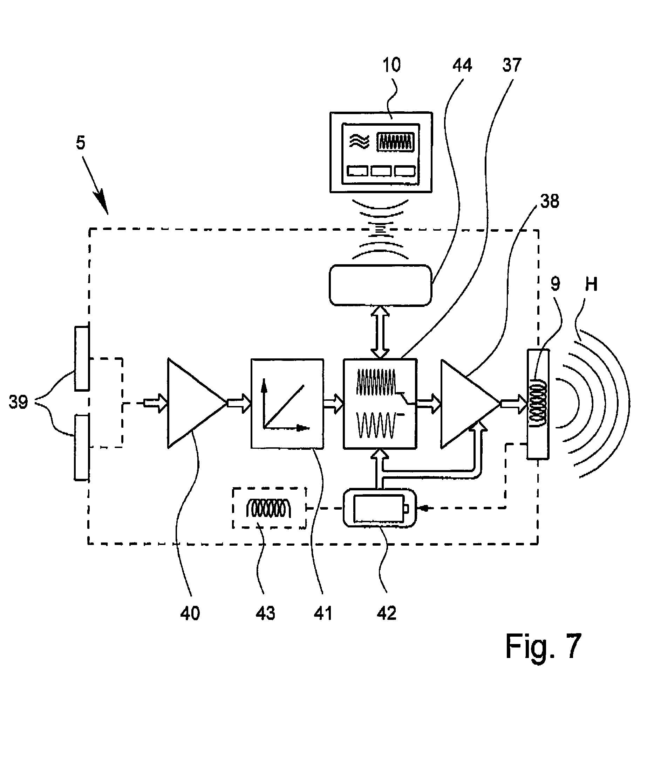

Time-varying magnetic field H preferably is generated by control device 5. Fig. 7 shows a schematic block diagram of control device 5 and programmer 10. Control device 5 preferably comprises sending means 9, in particular a coil, for generating time-varying magnetic field H. In particular, control 5 is adapted to generate time-varying magnetic field H of different transmission characteristics, in particular of transmission characteristics allowing for selectively activating high-energy and low-energy modes of a corresponding stimulation device 2. Control device 5 in Fig. 7 comprises a signal generator 37, in particular adapted for generation of signals with different transmission characteristics or corresponding thereto, in particular frequencies. These signals optionally can be amplified by amplifier 38 and preferably are transmitted by means of the time-varying magnetic field H, in particular via sending means 9. Thus, control device 5 can control stimulation device 2 to generate high-energy and/or low-energy stimulation impulses 3. Alternatively or additionally, control device 5 can synchronize generation and/or delivery of stimulation impulses 3.

Control device 5 can be implanted or placed outside the body 8. It can be programmed by external programmer 10, in particular wherein parameters can be configured defining particular transmission characteristics to be used.

Optionally, control device 5 can comprise electrodes 39 for sensing, e.g. an electrical behaviour of heart 11. The sensed signal can be amplified by amplifier 40 and/or processed by processor 41 which preferably is adapted to control signal generator 37. For example, a signal corresponding to the heart activity is received by electrodes 39 and processed by processor 41, which detects an anomaly. If this anomaly can be treated by means of low-energy stimulation impulses 3, signal generator 37 is controlled to generate a signal corresponding to a transmission characteristic of the time-varying magnetic field H leading to activation of generation and delivery of one or more low-energy stimulation impulses 3 by stimulation device 2. However, if processor 41 detects major problems that cannot be treated by means of low-energy impulses 3, processor 41 controls signal generator 37 to generate a signal corresponding to a time-varying magnetic field H of a transmission characteristic for generating a high-energy stimulation impulse 3 by stimulation device 2. The respective signals generated by signal generator 37 preferably are amplified and transmitted via sending means 9 forming corresponding time-varying magnetic field H.

Control device 5 preferably further comprises an energy store 41, in particular a rechargeable battery, which preferably is adapted to store energy of at least 1.5 times the energy needed for delivery of a high-energy stimulation impulse 3. In particular, energy store 42 stores more than 20 J, in particular more than 30 J. Energy store 42 can be charged by programmer 10, which preferably is adapted to communicate with a transceiver 44 of control device 5 for programming it and/or to provide control device 5 with energy for charging.

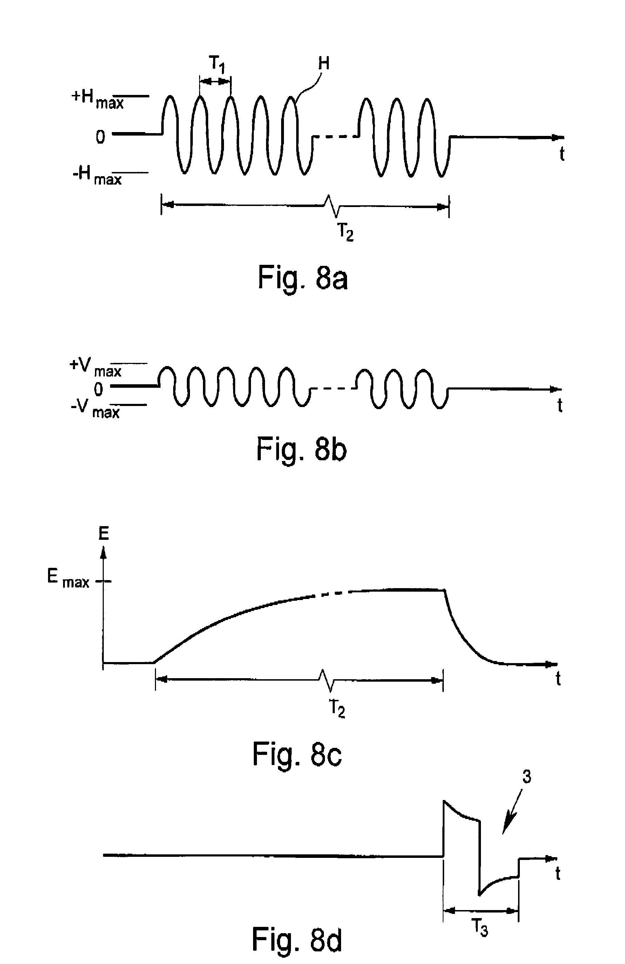

Fig. 8a shows a time-varying magnetic field H with amplitude Hₘₐₓ and with a frequency f = 1/T₁. Time-varying magnetic field H varies over a time span **T**₂ in particular forming a magnetic field burst. Fig. 8b shows a voltage with amplitude Vₘₐₓ which is induced to stimulation device 2 by means of time-varying magnetic field H of Fig. 8a. This voltage can be used for charging energy storing means 22. Energy E stored in energy storing means 22 is depicted in fig. 8c. This energy E can be used for generating stimulation impulse 3 shown in fig. 8d. This stimulation impulse can have a duration T₃, e.g. of more than 1 ms, preferably more than 5 ms, in particular more than 10 ms and/or less than 100 ms, preferably less than 50 ms, in particular less than 30 ms. Further, stimulation impulse 3 shown in fig. 8d comprises a zero-crossing. However, stimulation impulse 3 can have different shapes as well.

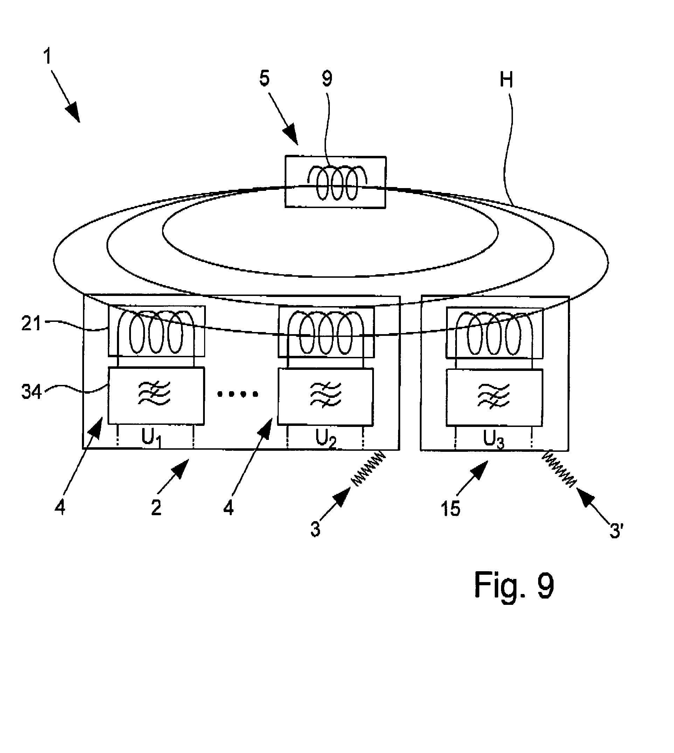

Fig. 9 is a simplified schematic view of stimulation system 1 with stimulation device 2, control device 5 and an additional, optional electrode device 15. Electrode device 15 preferably is configured as a wireless unit and is adapted to be supplied with energy exclusively in a wireless manner by means of time-varying magnetic field H. Wireless electrode device 15 preferably does not comprise any error-prone cable or lead. By supplying electrode device 15 with energy by means of time-varying magnetic field H, there is no demand for a battery or the like. Thus, a compact size can be achieved and lifetime of electrode device 15 is not limited by an energy storing device. In particular, electrode device 3 is passive, i.e. electrode device 15 receives at least the amount of energy by means of time-varying magnetic field H, that is delivered by electrode device 15, in particular by means of stimulation impulse 3'. Electrode device 15 preferably can be implanted close to or inside heart 11.

Stimulation device 2 and/or electrode device 15 preferably are adapted to deliver stimulation impulses 3, 3' comprising a sequence of pulses or oscillations.

Electrode device 15 preferably comprises an energy buffer, in particular with a capacity of less than 50 µF, preferably less than 10 µF, in particular less than 1 µF. Alternatively or additionally, electrode device 15 is adapted to buffer a total amount of energy for delivery of a maximum of ten low-energy electrical impulses 3', preferably two, in particular one low-energy electrical stimulation impulses 3'.

Electrode device 15 and/or stimulation device 2 can comprise delay means for delaying generation or delaying deliver of stimulation impulse 3, 3'. Electrode device 15 and/or stimulation device 2 can comprise receiving means adapted to receive and/or to reject commands and/or energy provided by means of time-varying magnetic field H of a particular transmission characteristic. In particular, filtering means can be provided therefor. A rectifier can be provided for rectifying energy received by means of time-varying magnetic field H. A pulse forming device can be provided by electrode device 15 for reshaping stimulation impulse 3' to be delivered, in particular in the time-domain.

Electrode device 15 can comprise a receiving means, in particular a coil, configured such that a pulse-like deduction voltage is generated when a minimum field strength of time-varying magnetic field **H** acting on electrode device 15 is exceeded. For this purpose, electrode device 15 can comprise a coil with a coil core which can cause an abrupt change in the magnetisation. In particular, a Wiegand wire is used as coil core.

According to one aspect of the present invention, one or more stimulation devices 2 and/or electrode devices 15 are adapted such that a time delay between two or more stimulation impulses 3, 3' of different stimulation devices 2 and/or electrode devices 15 can be obtained and/or controlled. Alternatively or additionally, one or more stimulation devices 2 and/or electrode devices 15 are adapted to be synchronized and/or supplied with energy by means of time-varying magnetic field H of different transmission characteristics. In particular, at least different modes of stimulation device 2 and/or stimulation device 2 and one or more of the electrode devices 15 are adapted to be selective to time-varying magnetic field H of different frequencies and/or polarities. Further, a time delay between two or more stimulation impulses 3, 3' of stimulation device 2 and/or electrode device 15 can be controlled and/or modified by means of time-varying magnetic field H of different transmission characteristics.

Stimulation device 2 preferably is adapted to distinguish different transmission characteristics of time-varying magnetic field H such that a high-energy stimulation impulse 3 can be generated if energy is received via a time-varying magnetic field H of a first transmission characteristic and a low-energy stimulation impulse 3 is generated if energy is received by means of time-varying magnetic field H of a different transmission characteristic. In particular, different elements 4 of stimulation device 2 can be influenceable by different transmission characteristics, in particular frequencies and/or by using filtering means 34. Alternatively or additionally, transmission characteristics of time-varying magnetic field H can be detected and stimulation device 2 decides based on this information which stimulation impulse 3 is to be generated and/or which modus is to be activated.

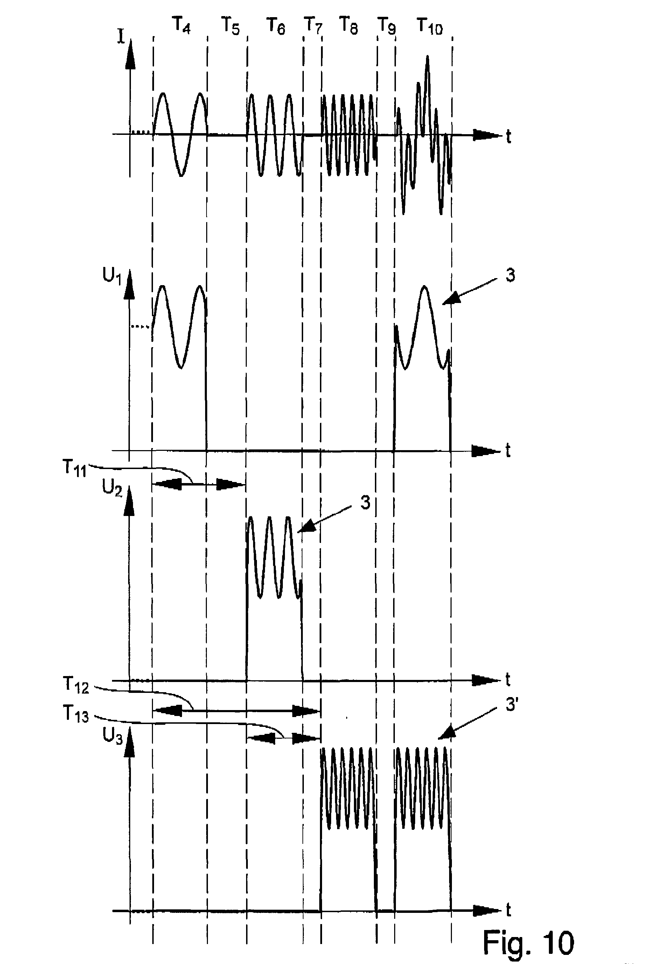

Electrode device 15 in Fig. 9 can be adapted to generate a low-energy stimulation impulse 3, in particular for pacing. It is preferred that generation and/or delivery of low-energy stimulation impulses 3 by stimulation device 2 and electrode device 15 are synchronized, in particular by means of time-varying magnetic field H of different transmission characteristics and/or by means of at least one delay means assigned to either stimulation device 2, electrode device 15 or both of them, preferably controlled by means of time-varying magnetic field H. This is discussed in further detail referring to Fig. 10a to 10d showing time diagrams.

Fig. 10 shows a current I corresponding to time-varying magnetic field H. Preferably, current I is provided to sending means 9, which converts this current I to corresponding time-varying magnetic field H.

In time spans T₄, T₆ and T₈, time-varying magnetic field H of first, second and third different transmission characteristics are generated, respectively. In the example shown, a first transmission characteristic is a low frequency, the second transmission charactistic is an intermediate frequency and the third transmission characteristic is a high frequency. These different transmission characteristics can be used for selectively activating and/or supplying stimulation device 2, in particular the low-energy mode and the high-energy mode of stimulation device 2, and/or electrode device 15.

In the example shown in Fig. 9, different filtering means 34 are provided, in particular a low pass filter, a band pass filter and a high pass filter. One or more first elements 4 of stimulation device 2 can be adapted by a low pass filter to selectively be sourced by time-varying magnetic field H provided in time span T₄, in particular causing an energy transfer and/or induction voltage U₁. This energy and/or voltage can be used for generating a low-energy stimulation impulse 3 by stimulation device 2, e.g. for pacing. A band pass filter can be used by stimulation device 2 for selectively accepting energy provided by means of time-varying magnetic field H in time T₆, in particular causing an energy transfer and/or induction voltage U₂. This energy can be used for generating a high-energy stimulation impulse 3 by stimulation device 2, e.g for defibrillation. Thus, different transmission characteristics, in particular frequencies, of time spans T₄ and T₆, can be used for activating high-energy and low-energy stimulation impulses 3, respectively, to be delivered by stimulation device 2, and/or corrsponding modes.

Electrode device 15, in the example shown, is adapted to selectively accept energy provided by means of time-varying magnetic field H in time span T₈, i.e. of the third transmission characteristic. In particular, energy is transferred to electrode device 15 and/or induction voltage U₃ is caused in electrode device 15. This allows for selectively activating electrode device 15 to generate a stimulation impulse 3', in particular a low-energy stimulation impulse 3' and/or for pacing.

In time span T₁₀, time-varying magnetic field H provides transmission characteristics of both time spans T₄ and T₈. Thus, time-varying magnetic field H in time span T₁₀ allows for activating low-energy mode of stimulation device 2 as well as for activating electrode device 15. These activations, i.e. generation and/or delivery of low-energy stimulation impulses 3, 3', can be synchronized to be delivered simultaneously or to be delivered with a particular time delay, in particular by control device 5. This allows for multi-site pacing, i.e. for stimulating heart 11 in a synchronized manner at different locations. Thus, stimulation efficiency can be increased.

In a preferred alternative, the transmission characteristic for activating high-energy mode sources elements 4 for low-energy mode as well. Thus, at least elements 4 assigned to the low-energy mode can be used either for the high-energy or the low-energy mode. Further, energy can be transferred to each element 4 and activating high-energy and/or low-energy mode can be controlled differently, in particular by filtering means 34 used for controlling selective activation of high-energy, low-energy mode and/or generation of respective stimulation impulses 3, e.g. by means of switch 36 and/or pulse-forming means 33.

In the example of Fig. 9, filtering means 34 are used for mode and/or device selection. However, alternatively or additionally, different transmission characteristics as discussed in the beginning can be used for selectively charging and/or for selectively activating modes and/or devices. Further, low-energy mode of stimulation device 2 as well as electrode device 15 can be supplied with energy and/or activated by means of the same transmission characteristic. This still allows for selectively activating generation of a high-energy stimulation impulse 3 and a low-energy stimulation impulse 3 and/or for multi-site pacing.

A further aspect of the present invention relates to a method for treating cardiac fibrillation or cardiac tachycardia, comprising implanting control device 5 at a subcutaneous implantation site to generate time-varying magnetic field H, preferably wherein control device 5 comprises sensing electrodes 39 that are configured to detect undesirable cardiac arrhythmias. It is further preferred that stimulation device 2 is implanted at a cardiac implantation site, wherein stimulation device 2 comprises one or more electrodes 16, 17 adapted to deliver defibrillation energy to cardiac tissue. Further, time-varying magnetic field H can be generated at the subcutaneous implantation site using control device 5, i.e. control device 5 can be implantable. Energy provided by time-varying magnetic field H preferably is received by stimulation device 2 at the cardiac implantation site. External programmer 10 can be used to program one or more settings of control device 5. Energy provided by means of the time-varying magnetic field H preferably is converted to electrical energy by stimulation device 2, in particular at a rate of 1 to 10 W, can be stored at a level to produce an appropriate amplitude, and delivered with an appropriate wave form (stimulation impulse 3) sufficient to treat the cardiac fibrillation or cardiac tachycardia.

According to a further aspect of the present invention, stimulation system 1 is adapted for treating cardiac fibrillation or cardiac tachycardia, the system comprising control device 5 comprising sensing electrodes 39 to detect undesirable cardiac arrhythmias. Stimulation system 1 further comprises implantable stimulation device 2 preferably comprising electrodes 16, 17 adapted to deliver defibrillation energy to the cardiac muscle tissue. Stimulation system 1 preferably further comprises external programmer 10 configured to program one or more settings of control device 5. It is particularly preferred that control device 5 and stimulation device 2 are adapted to transmit and receive energy by means of time-varying magnetic field H, respectively, providing both defibrillation energy and signal information to stimulation device 2 sufficient to provide cardiac defibrillation, termination of cardiac tachycardia and, preferably, for cardiac pacing. Stimulation device 2 preferably is configured to convert received energy to electrical energy at a rate of 1 to 10 W.

Stimulation device 2, in particular one or more of the elements 4, is or are adapted for receiving energy by means of time-varying magnetic field H and for converting this energy to electrical energy. This electrical energy can be accumulated and/or stored by stimulation device 2, in particular by one ore more of the elements 4. Stimulation device 2 further preferably comprises means to generate an appropriate wave form from the stored electrical energy to defibrillate the cardiac tissue. Stimulation device 2 further preferably comprises electrodes 16 adapted to deliver the electrical energy and/or wave form (stimulation impulse 3) to cardiac tissue.

According to one aspect of the present invention, stimulation device 2 has distal and proximal ends, where the distal end of stimulation device 2 is adapted to be secured in the right ventricular apex of heart 10, and the proximal end is adapted to be located above heart 11. In particular, one of the electrodes 16 is adapted to be located near the right ventricular apex of heart 11, another one of electrodes 16 is adapted to be located within the brachiocephalic subclavian, or a vein generally superior to heart 11.

According to one further aspect of the present invention, control device 5 comprises a power source, in particular energy store 42, as well as control and timing circuitry to detect undesirable cardiac arrhythmias and provide a defibrillation output signal, particularly preferably a transmission characteristic of time-varying magnetic field H. Control device 5 further can comprise means for converting the defibrillation output signal to time-varying magnetic field H comprising a corresponding information, in particular transmission characteristic, and is adapted for transmitting time-varying magnetic field H to stimulation device 2.

Control device 5, in particular control circuitry or processor 41, is configured to process an ECG signal from the sensing electrodes 39, wherein electrodes 39 are adapted to be located on the exterior surface of control device 5 and in contact with tissue of body 8.

A further aspect of the present invention relates to a method for treating cardiac fibrillation or cardiac tachycardia and stimulating cardiac muscle. Control device 5 is implanted at a subcutaneous implantation site to generate time-varying magnetic field H, wherein control device 5 comprises electrodes 39 for sensing that are configured to detect undesirable cardiac arrhythmias. Stimulation device 2 is implanted at a cardiac implantation site, wherein stimulation device 2 comprises one or more electrodes 16, 17 preferably adapted to be in direct contact with cardiac tissue and for delivering defibrillation or stimulation energy to cardiac muscle tissue. Time-varying magnetic field H is generated at the subcutaneous implantation site using control device 5. Energy provided by means of time-varying magnetic field H is received at the cardiac implantation site using stimulation device 2. External programmer 10 can be used to program one or more settings of control device 5. Energy provided by means of time-varying magnetic field H preferably is converted by stimulation device 2 to electrical energy at a rate of more than 1 W and/or less than 10 W, preferably wherein the electrical energy is suitable for either cardiac defibrillation or for treating cardiac tachycardia or for cardiac muscle stimulation based on both energy and signal information encoded in the generated time-varying magnetic field H.

According to a particular aspect of the present invention, energy provided by means of the time-varying magnetic field H is received at least to different cardiac sites. In particular, a predetermined configuration causes stimulation of at least two different sites simultaneously.

A further aspect of the present invention relates to providing energy to stimulation device 2 sufficient for accomplishing one of either stimulation or defibrillation. Signal information or control commands can be provided to stimulation device 2, in particular by means of a transmission characteristic or frequency, sufficient to perform cardiac defibrillation or termination of cardiac tachycardia, in particular wherein stimulation device 2 is configured to convert received energy to electrical energy at a rate of 1 to 10 W.

Stimulation device 2 can be adapted to receive energy by means of time-varying magnetic field H and to generate an alternating current signal therefrom. Stimulation device 2 further can comprise means for converting the alternating current signal to a direct current signal and further converting the direct current signal into stimulation energy of (defibrillation) stimulation impulse 3. Further, stimulation device 2 can comprise means for deciding whether the energy provided by means of the time-varying magnetic field H is intended to stimulate or defibrillate. In particular, stimulation device 2 is adapted for detecting a transmission characteristic of time-varying magnetic field H and for determining whether delivery of a high-energy stimulation impulse 3 and/or a low-energy stimulation impulse 3 is intended. If so, stimulation device 2 preferably is adapted to generate a respective stimulation impulse 3.

Stimulation device 2 preferably comprises means for applying stimulation energy, i.e. the stimulation impulse 3, to electrodes 16, 17 which preferably are adapted to deliver stimulation energy, i.e. the stimulation impulse 3, to myocardial tissue. It is further preferred that energy for generation of the stimulation impulse 3 can be accumulated and/or supported by stimulation device 2. Further, stimulation device 2 can comprise means to generate stimulation impulse 3 of an appropriate wave form to defibrillate the cardiac tissue utilizing the stored defibrillation energy. The electrodes 16,17 preferably are adapted to deliver the defibrillation wave form to myocardial tissue.

Stimulation device 2 can be adapted to be secured in the right ventricular apex of heart 11, and the proximal end of stimulation device 2 can be adapted to be located above heart 11, preferably wherein the proximal end is adapted to be located above heart 11 in the superior vena cava.

Control device 5 can comprise a power source (energy store 42), control circuitry and timing circuitry to provide a pacing signal and to detect undesirable cardiac arrhythmias and provide a pacing and defibrillating output signal as well as means for converting the output signal to a time-varying magnetic field H, in particular of a transmission characteristic corresponding to the detection result, and control device 5 comprises means for transmitting the time-varying magnetic field H to stimulation device 2. Control device 5 optionally can act as electrode device 15 and/or stimulation device 2 as well.

A time-varying magnetic field can comprise one or more frequencies, preferably higher than 100 kHz, in particular higher than 500 kHz and/or lower than 50 MHz, in particular lower than 10 MHz. Energy can be transmitted to stimulation device 2 by continuously varying magnetic field H, preferably less than 20 s, more preferably less than 10 s, in particular less than 5 s.

Depending on the configuration, stimulation device 2 can also be used independently of control device 5. For example, it is possible in principle that stimulation device 2 can be supplied with energy and/or controlled by another device, optionally even by a nuclear spintomograph or the like, in particular with suitable matching. Thus, further possible uses are obtained which go substantially beyond the possible uses on conventional pacemakers, defibrillators or other stimulation systems. In particular, stimulation device 2 can be used for electrolysis or for electroanalysis, for instance while performing a nuclear resonance spectroscopy or the like. Further, control device 5 can be used independently as well, e.g. for selectively supplying other components or controlling them by means of the time-varying magnetic field H of different transmission characteristics.

### Reference Numerals

- 1: stimulation system
- 2: stimulation device
- 3: stimulation impulse
- 4: element
- 5: control device
- 6: skin
- 7: thoracic cage
- 8: body
- 9: sending means
- 10: programmer
- 11: heart
- 12: ventricle
- 13: atrium
- 14: superior vena cava
- 15: electrode device
- 16: electrode
- 17: electrode
- 18: anchor
- 19: cover
- 20: interface
- 21: receiving means
- 22: energy storing means
- 23: cap
- 24: thread
- 25: beat
- 26: charging means
- 27: shell
- 28: inner lead
- 29: outer lead
- 30: insulator
- 31: mounting means
- 32: converter
- 33: pulse forming means
- 34: filtering means
- 35: triggering means
- 36: activation means
- 37: signal generator
- 38: amplifier
- 39: electrode
- 40: amplifier
- 41: processor
- 42: energy store
- 43: charging coil
- 44: transceiver
- C: capacity
- H: magnetic field
- Hₘₐₓ: amplitude
- I: current
- T₁ to T₁₃: time span
- U₁ to U₃: induction voltage
- Vₘₐₓ: amplitude

## Claims

1. Implantable stimulation device (2) for generating electrical stimulation impulses (3), wherein the stimulation device (2) is adapted to be controlled and/or supplied with energy in an exclusively wireless manner by means of a time-varying magnetic field (H),
wherein the stimulation device (2) is configured for generating high-energy stimulation impulses (3) for defibrillation and low-energy stimulation impulses (3) for pacing, wherein a high-energy stimulation impulse (3) is generated when the stimulation device (2) is supplied with energy or controlled by means of the time-varying magnetic field (H) of a first transmission characteristic and a low-energy stimulation impulse (3) is generated when the stimulation device (2) is supplied with energy or controlled by means of the time-varying magnetic field (H) of a different second transmission characteristic, and/or
wherein the stimulation device (2) comprises a multiplicity of linked elements (4), wherein at least two or all of the elements (4) are adapted to store energy and/or to be supplied with energy by means of the time-varying magnetic field (H).

2. Implantable stimulation device of claim 1, **characterized in that** at least two of the elements (4) each comprises an energy storing means (22), preferably configured to store more than 50 mJ, in particular more than 100 mJ and/or less than 2 J, in particular less than 1 J.

3. Implantable stimulation device of claim 1 or 2, **characterized in that** at least two of the elements (4) each comprises a receiving means (21) which is adapted for receiving energy in an exclusively wireless manner by means of the time-varying magnetic field (H) and for converting this energy to electrical energy, preferably with an output power rating of more than 50 mW, in particular more than 100 mW and/or less than 2 W, in particular less than 1 W, and/or wherein the stimulation device (2) is adapted to be charged with more than 500 mW, preferably more than 1 W and/or less than 20 W, preferably less than 10 W.

4. Implantable stimulation device of any one of the preceding claims, **characterized in that** the linked elements (4) form a joint energy storing means (22) and/or receiving means (21) of the stimulation device (2), preferably wherein the amount of energy storable by the stimulation device (2) and/or the power rating of energy transferable to the stimulation device (2) is or are variable by means of the number of elements (4) the stimulation device (2) comprises.

5. Implantable stimulation device of claim 1, **characterized in that** the stimulation device (2) is flexible by means of the multiplicity of linked elements (4), preferably wherein the elements (4) are shaped as tube-like sections, and/or wherein face sides of adjacent elements (4) are hinged to each other.

6. Implantable stimulation device of any one of the preceding claims, **characterized in that** stimulation device (2) is elongately shaped by means of the multiplicity of linked elements (4), in particular with a length greater than 10 cm, preferably greater than 12 cm, in particular greater than 15 cm, and/or smaller than 25 cm, preferably smaller than 22 cm, in particular smaller than 18 cm, and/or with a diameter smaller than 5 mm, preferably smaller than 4 mm, in particular smaller than 3 mm, and/or greater than 0.5 mm, preferably greater than 1 mm, in particular greater than 1.5 mm.

7. Implantable stimulation device of any one of the preceding claims, **characterized in that** the stimulation device (2) provides a high-energy mode and a low-energy mode, wherein the energy delivered by the stimulation device (2) by each stimulation impulse (3) in the high-energy mode is higher than energy delivered in each stimulation impulse (3) in the low-energy mode, preferably at least 50 or 100 times, in particular 200 times, higher.

8. Implantable stimulation device of claim 7, **characterized in that** the stimulation device (2) is adapted to selectively activate the high-energy or the low-energy mode, respectively, if energy is transmitted using a time-varying magnetic field (H) of a first or a different second transmission characteristic, preferably of a first and a different second frequency.

9. Implantable stimulation device of any one of the preceding claims, **characterized in that** the stimulation device (2) comprises an anchor for securely fastening it which is adapted for delivering an electrical impulse (3) additionally.

10. Implantable stimulation device of any one of the preceding claims, **characterized in that** the stimulation device (2) comprises a first pair of electrodes (16, 17) having a shorter distance to one another which is configured to deliver low-energy stimulation impulses (3), in particular for pacing and/or to a tissue of a cardiac muscle, and a second pair of electrodes (16, 17) having a larger distance to one another which is configured to deliver high-energy stimulation impulses (3), preferably for defibrillation and/or to a tissue of a cardiac muscle.

11. Stimulation system (1) comprising a stimulation device (2) adapted for delivering an electrical stimulation impulse (3) of any one of the preceding claims, wherein the stimulation system (1) further comprises a control device (5) adapted for controlling the stimulation device (2) and/or supplying the stimulation device (2) with energy in an exclusively wireless manner by means of a time-varying magnetic field (H).

12. Stimulation system of claim 11, wherein the control device (5) and the stimulation device (2) are adapted to transmit and receive energy by means of the time-varying magnetic field (H), respectively, which provides both energy and control information to the stimulation device (2) sufficient for generation of the electrical stimulation impulse (3) by the stimulation device (2), and/or with power transfer capability of more than 500 mW, preferably higher than 1 W and/or lower than 20 W, preferably lower than 10 W.

13. Stimulation system of claim 11 or 12, **characterized in that** the control device (5) comprises sensing electrodes (16, 17) configured to detect cardiac arrhythmias, preferably wherein the control device (5) is adapted to automatically transmit energy by means of the time-varying magnetic field (H) of a particular transmission characteristic, in particular frequency, when a cardiac arrhythmia is detected and the stimulation device (2) is adapted to enter high-energy mode and/or to deliver a defibrillation impulse (3) if the stimulation device (2) receives energy by means of the time-varying magnetic field (H) of this transmission characteristic, in particular frequency.

14. Stimulation system of any one of claims 11 to 13, **characterized in that** the system (1) further comprises an external programmer (10) configured to program one or more settings of the control device (5), in particular corresponding to transmission characteristics for the time-varying magnetic field (H) to be used for entering high-energy mode and/or low-energy mode of the stimulation device (2).

15. Method for controlling a stimulation device (2) for generating high-energy and low-energy electrical impulses (3), wherein the stimulation device (2) is controlled and/or supplied with energy in an exclusively wireless manner by means of a time-varying magnetic field (H), such that the stimulation device (2) generates a high-energy electrical stimulation impulse (3) when supplied with energy or controlled by means of a time-varying magnetic field (H) of a first transmission characteristic and a low-energy electrical stimulation impulse (3) when supplied with energy or controlled by means of the time-varying magnetic field (H) of a different second transmission characteristic.
